# EUROPÄISCHE PATENTSCHRIFT

(11) **EP 2 195 259 B1**
(45) Veröffentlichungstag und Bekanntmachung des Hinweises auf die Patenterteilung: **09.08.2017**
(21) Anmeldenummer: 08836158.9
(22) Anmeldetag: 29.09.2008
(51) Int. Cl.: B65D 83/14, B05B 11/00, B65D 83/30, A61M 15/00

(54) **SPRÜHDOSE MIT AUSGABEROHR**
SPRAY CAN COMPRISING A DISCHARGE TUBE
CANETTE DE PULVÉRISATION DOTÉE D'UN TUBE DE SORTIE

(30) Priorität: 02.10.2007 DE 202007013747 U
(43) Veröffentlichungstag der Anmeldung: 16.06.2010
(73) Patentinhaber: Sulzer Mixpac AG, 9469 Haag (Rheintal) (CH)
(72) Erfinder: SOGARO, Alberto, C., 61476 Kronberg (DE)
(74) Vertreter: Manitz Finsterwald Patentanwälte PartmbB
(86) Internationale Anmeldenummer: PCT/DE2008/001610
(87) Internationale Veröffentlichungsnummer: WO 2009/043342

(56) Entgegenhaltungen:
- EP-A- 1 088 772
- WO-A-2006/105923
- WO-A-2007/009282
- WO-A1-97/03756
- CH-A- 378 802
- DE-A1- 2 338 800
- DE-U1-202006 012 416
- GB-A- 805 203
- JP-A- 7 285 585
- US-B1- 6 783 037

## Beschreibung

Die Erfindung betrifft eine Sprühdose, die einen Düsenkörper zur Aufnahme eines zu versprühenden Stoffes und einen Sprühkopf aufweist, der auf ein an der Oberseite des Düsenkörpers angeordnetes Dosenventil aufgesteckt ist und der mit einem Ausgaberohr versehen ist.

Eine derartige Sprühdose ist aus der Praxis bekannt und dient zur Ausgabe und zum Zerstäuben flüssiger pharmazeutischer Produkte. Die Dose bzw. der Behälter ist mit einem Entnahme- und/oder Dosierorgan ausgestattet, das ein Ventil sein kann. Insbesondere kann die Sprühdose zur Applikation des pharmazeutischen Produktes im Mund und Rachen eines Patienten eingesetzt werden. Die bekannte Sprühdose umfasst einen Sprühkopf, der auf das Dosierventil aufgesetzt ist und mit einem Rohrabschnitt versehen ist, auf den ein Endbereich eines Ausgaberohrs über eine Drehverbindung aufgesteckt ist. Das Ausgaberohr ist auf dem Rohrabschnitt derart gelagert, dass das rechtwinklig ausgebildete Ausgaberohr auf dem Rohrabschnitt zur anforderungsgemäßen Ausrichtung des Ausgaberohrs gedreht werden kann.

Bei der bekannten Sprühdose besteht der Nachteil, dass das Ausgaberohr nur mit hohem Kraftaufwand und gegebenenfalls nur unter Beschädigung des Ausgaberohrs wechselbar ist. Ein Wechseln des Ausgaberohrs kann aber erforderlich sein, damit bestehenden hygienischen Anforderungen Genüge geleistet werden kann.

Der Erfindung liegt die Aufgabe zugrunde, eine Sprühdose zu schaffen, die ein Ausgaberohr an einem Sprühkopf aufweist, welches in einfacher Weise und mit geringem Kraftaufwand austauschbar ist.

Diese Aufgabe ist erfindungsgemäß durch die Sprühdose mit den Merkmalen des Patentanspruchs 1 bzw. 7 gelöst. Der Oberbegriff des Anspruchs 1 ist in US 6,783,037 und der des Anspruchs 7 in JP 7285585 offenbart. Erfindungsgemäß wird mithin eine Sprühdose vorgeschlagen, die einen Dosenkörper zur Aufnahme eines zu versprühenden Stoffes und einen Sprühkopf aufweist, der auf ein an der Oberseite des Dosenkörpers angeordnetes Dosenventil aufgesteckt ist und der mit einem Ausgaberohr versehen ist. Es ist ein Adapter vorgesehen, der den Sprühkopf mit dem Ausgaberohr verbindet, wobei das Ausgaberohr über eine zerstörungsfrei lösbare Steck- und/oder Schraubverbindung mit dem Adapter verbunden ist.

Durch die erfindungsgemäße Auslegung einer Sprühdose ist es möglich, in einfacher Weise in kurzer Zeit das Ausgaberohr zu wechseln, um beispielsweise bei Verwendung derselben Sprühdose bei verschiedenen Patienten in deren Mund bzw. Rachenbereich stets ein antiseptisches bzw. hygienisch den bestehenden Anforderungen genügendes Aüsgaberohr verwenden zu können. Es wird einfach die Steck- und/oder Schräubverbindung gelöst und ein neues Ausgaberohr auf den Adapter aufgesetzt.

Die Sprühdose nach der Erfindung eignet sich insbesondere zum Applizieren eines Mattierungsmittels auf einen Zahn bzw. Zahnstumpf, der mittels eines Videosystems abgescant werden soll, oder eines Okklusionsprüfmittels.

Bei einer bevorzugten Ausführungsform der Sprühdose nach der Erfindung umfasst die Steckverbindung einen Außenkonus, der an dem Adapter oder an dem Ausgaberohr ausgebildet ist und der mit einem Innenkonus des Ausgaberohrs bzw. des Adapters zusammenwirkt, so dass zwischen, dem Adapter und dem Ausgaberohr eine Presspassung vorliegt. Diese Art der Verbindung entspricht dem so genannten Luer-Prinzip.

Um zu verhindern, dass sich das Ausgaberohr bei Benutzung der Sprühdose in ungewollter Weise von dem Adapter löst, ist bei einer bevorzugten Ausführungsform der Sprühdose nach der Erfindung eine Sicherungseinrichtung vorgesehen, die das Ausgaberohr an dem Adapter hält und zum Trennen des Ausgaberohrs von dem Adapter lösbar ist. Erfindungsgemäß ist die Sicherungseinrichtung eine an dem Adapter gelagerte Überwurfmutter, die mit einem Gegenstück zusammenwirkt, das an dem Ausgaberohr angeordnet ist. Insbesondere ist das Gegenstück ein Endstück des Ausgaberohrs bzw. ein an dem Endstück des Ausgaberohrs ausgebildetes Element, wie eine radiale Lasche zum Angriff eines Gewindes.

Vorzugsweise weist der Adapter eine Ringnut auf, in der die Überwurfmutter unverlierbar gelagert ist.

Die Ringnut kann so ausgebildet sein, dass die Überwurfmutter in axialer Richtung auf dem Adapter verschiebbar ist. Beim Lösen der Überwurfmutter von dem Gegenstück erfährt diese dann einen axialen Versatz.

Die Ringnut kann eine derartige Breite aufweisen, dass ihr dem Ausgaberohr abgewandter Rand als hinterer Anschlag für die Überwurfmutter beim Lösen des Ausgaberohrs von dem Adapter dient. Zweckmäßigerweise schlägt die Überwurfmutter kurz vor Erreichen ihrer Freigabestellung diesen Anschlag. Ein dann erfolgendes Weiterdrehen der Überwurfmutter bewirkt einen Versatz des Ausgaberohrs relativ zu dem Adapter, so dass ein Presssitz zwischen diesen beiden Elementen durch den von dem Innengewinde der Überwurfmutter auf das Gegenstück des Ausgaberohrs ausgeübten Druck gelöst wird. Das Ausgaberohr kann dann einfach von dem Adapter abgenommen werden.

Denkbar ist es natürlich auch, dass eine Überwurfmutter der beschriebenen Art an dem Ausgaberohr vorgesehen ist und das Ausgaberohr ein Außenkonus und an dem Adapterstück ein Innenkonus ausgebildet ist. Das Gegenstück ist dann ebenfalls an dem Adapter ausgebildet, beispielsweise in Form eines Ringbunds, der zur Ausbildung von radial abstehenden Laschen mindestens eine Ausnehmung aufweist, in die ein Gewinde der Überwurfmutter eingreifen kann.

Das Endstück bzw. das Gegenstück kann entweder auf einen Rohrabschnitt des Ausgaberohrs aufgesetzt sein oder auch einstückig mit diesem gefertigt sein.

Bei einer alternativen Ausführungsform der Sprühdose nach der Erfindung weist das Ausgaberohr an seinem dem Adapter zugewandten Ende mindestens eine Rastlasche auf, die mit einem Rastelement in eine Ausnehmung des Adapters eingreift.

Bei einer konstruktiv einfach umzusetzenden Ausführungsform ist die Rastlasche durch axiale Schlitze im Endbereich des Ausgaberohrs gebildet.

Um das Ausgaberohr positionsgenau mit dem Adapter verbinden zu können, ist das Ausgaberohr vorzugsweise in einen Kanal des Adapters eingesteckt, wobei der Adapter eine Führungsbahn für das Rastelement aufweist.

Um das Lösen des Ausgaberohrs von dem Adapter einfach zu gestalten, weist die Rastlasche bei einer bevorzugten Ausführungsform ein in radialer Richtung vorstehendes Betätigungselement, beispielsweise in Form eines Tasters auf. Das Betätigungselement ist beim Einstecken des Ausgaberohrs in dem Adapter in der Führungsbahn geführt. Das Betätigungselement stellt mithin ebenfalls eine Führungseinrichtung dar, die ein Verkanten des Ausgaberohrs in dem Adapter bei dessen Einstecken in den Adapter verhindert.

Der Kanal des Adapters, in den das Ausgaberohr eingesteckt wird, kann mit einem Dichtmaterial, beispielsweise mit einer gummielastischen Schicht, z.B. aus PTE, versehen sein. Eine solche Schicht erhöht die Dichtigkeit des Gesamt systems.

Bei einer weiteren alternativen Ausführungsform der Sprühdose nach der Erfindung ist das Ausgaberohr mittels einer Muffe an dem Adapter fixiert. Diese Ausführungsform ist kostengünstig realisierbar.

Bei einer bevorzugten Ausführungsform der Sprühdose nach der Erfindung ist der Adapter über eine Presspassung mit dem Sprühkopf verbunden. Die Presspassung sollte so ausgelegt sein, dass der Adapter nicht werkzeugfrei aus dem Sprühkopf gezogen werden kann. Der Adapter und ein Gehäuse des Sprühkopfs können aber auch einstückig hergestellt sein.

Insbesondere zum Zerstäuben eines Stoffes im Mund- bzw. Rachenraum eines Patienten ist es vorteilhaft, wenn das Ausgaberohr an seinem dem Sprühkopf abgewandten Ende mit einem Düsenkopf versehen ist. Dieser kann einen üblichen, aus der Praxis bekannten Aufbau haben. Der Düsenkopf kann zur Ausrichtung des Ausgabestrahls drehbar auf dem Ausgaberohr gelagert sein.

Weitere Vorteile und vorteilhafte Ausgestaltungen des Gegenstandes der Erfindung sind der Beschreibung, der Zeichnung und den Patentansprüchen entnehmbar.

Zwei Ausführungsbeispiele einer Sprühdose nach der Erfindung sind in der Zeichnung schematisch vereinfacht dargestellt und werden in der nachfolgenden Beschreibung näher erläutert. Es zeigt
- Fig. 1: eine Sprühdose mit einem Sprühkopf und einen in erfindungsgemäßer Weise ausgebildeten Adapter/Ausgaberohr-System vor dem Aufsetzen des Ausgaberohrs;
- Fig. 2: eine Fig. 1 entsprechende Ansicht, während des Aufsetzens des Ausgaberohrs auf den Adapter;
- Fig. 3: ebenfalls eine Fig. 1 entsprechende Ansicht, jedoch beim Sichern des Ausgaberohrs auf dem Adapter;
- Fig. 4: einen Längsschnitt durch den Adapter;
- Fig. 5: eine Seitenansicht eines Endstücks des Ausgaberohrs;
- Fig. 6: eine Stirnansicht des Endstücks;
- Fig. 7: eine Seitenansicht einer Überwurfmutter zur Sicherung an dem Endstück;
- Fig.: 8 eine Stirnansicht der Überwurfmutter;
- Fig. 9: eine Übersichtsdarstellung einer zweiten Ausführungsform im zerlegten Zustand; und
- Fig. 10: die in Fig. 9 dargestellte Ausführungsform im montierten Zustand.

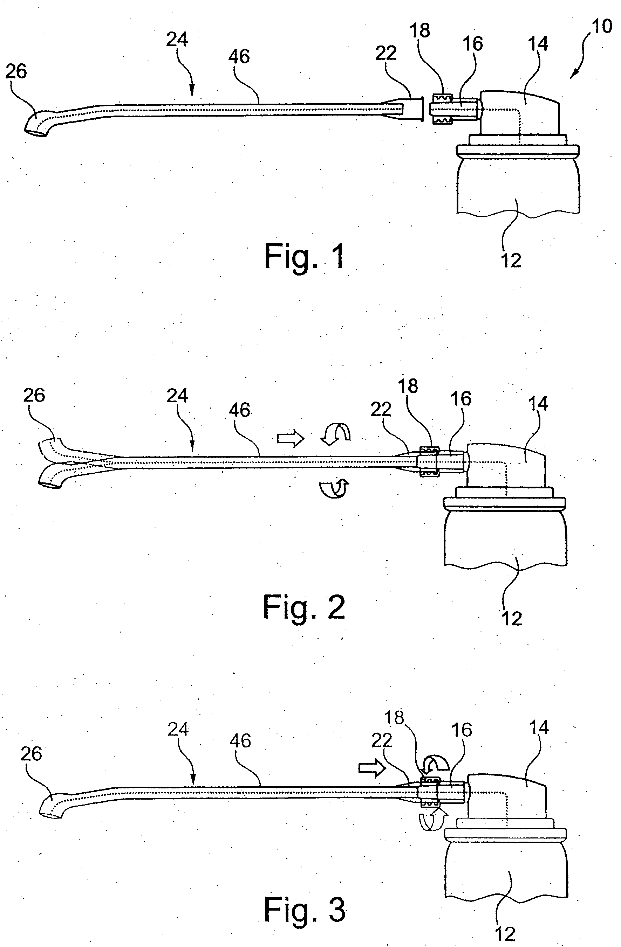

In den Figuren 1 bis 8 ist eine Sprühdose 10 dargestellt, die einen zylindrischen Dosenkörper 12 aufweist, auf den oberseitig bzw. stirnseitig auf ein nicht näher dargestelltes Dosenventil ein Sprühkopf 14 aufgesetzt ist.

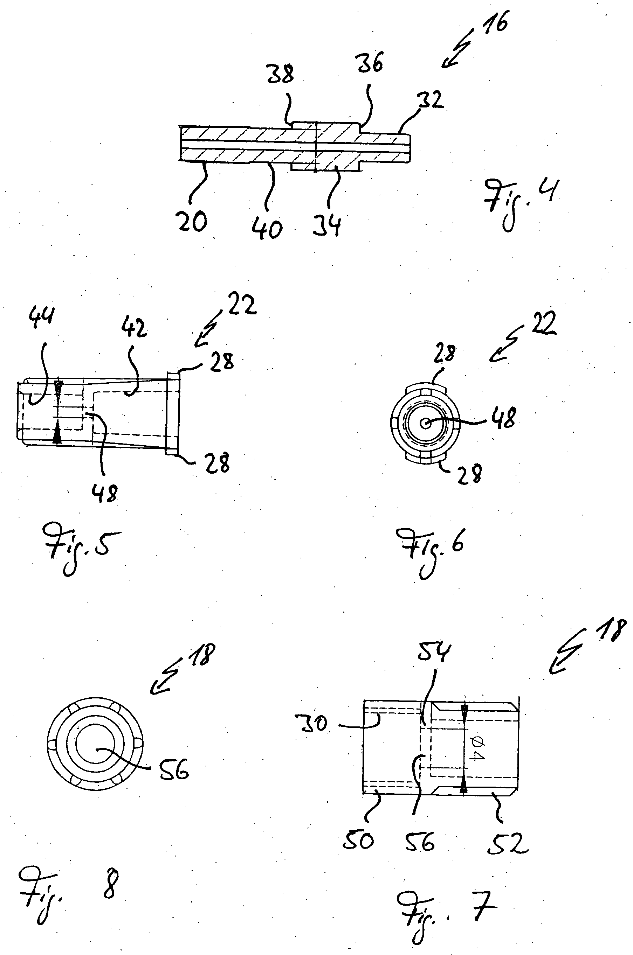

Der Sprühkopf 14, der zur Betätigung, d.h. zum Öffnen des Dosenventils, in Richtung des Dosenkörpers 12 niedergedrückt wird, weist einen in radialer Richtung vorstehenden Adapter 16 auf, der in eine radiale Öffnung des Sprühkopfs 14 über eine Presspassung eingesteckt ist und in Fig. 4 detailliert dargestellt ist.

Auf dem Adapter 16 ist eine Überwurfmutter 18 drehbar gelagert.

An seinem dem Sprühkopf abgewandten Endbereich 20, der einen Außenkonus bildet, kann, wie in den Figuren 2 und 3 dargestellt ist, ein Endstück 22 eines Ausgaberohr 24 aufgesteckt werden, das an seinem dem Endstück 22 abgewandten Ende mit einem Düsenkopf 26 versehen ist, der drehbar gelagert ist und bei Benutzung der Sprühdose 10 eine Zerstäubung des auszubringenden Stoffes bewirkt.

Zur Sicherung des Ausgaberohrs auf dem Adapter 16 kann die Überwurfmutter 18 mit dem Endstück 22 des Ausgaberohrs 24 verschraubt werden. Hierzu weist das Endstück 22 stirnseitig einen Ringbund bzw. zwei um 180° zueinander versetzte, radial vorstehende Laschen 28 auf, die mit einem Innengewinde 30 der Überwurfmutter 18 zusammenwirken.

Dadurch, dass die Überwurfmutter frei drehbar auf dem Adapter 16 ist, kann das Ausgaberohr zur Ausrichtung des Düsenkopfs 26 frei auf dem Adapter 16 gedreht werden. Erst nach der gewünschten Orientierung des Düsenkopfs 26 wird die Überwurfmutter 28 durch Angriff von deren Innengewinde 30 an den Laschen 28 an dem Endstück 22 des Ausgaberohrs 24 gesichert (vgl. Fig. 3).

Der Adapter 16, der in Fig. 4 in Alleinstellung dargestellt ist, bildet ein Röhrchen mit einem einheitlichen Innendurchmesser von beispielsweise 1 mm, das einerseits den konischen Endbereich 20 aufweist und andererseits mit einem zylindrischen Endbereich 32 versehen ist, der in Presspassung in der entsprechenden Öffnung des Sprühkopfs 14 eingesteckt ist. In einem mittleren Bereich des Adapters 16 ist ein Ringbund 34 ausgebildet, der mit einer Stirnfläche 36 einen Anschlag für das Gehäuse des Sprühkopfs 14 bildet und mit seiner zweiten Stirnfläche 38 einen Anschlag für die Überwurfmutter 18 bildet, welche in einer Ringnut 40 bzw. Einschnürung 40, die im vorliegenden Fall eine Länge von etwa 5 mm hat, verschiebbar gelagert ist.

In Montagestellung sitzt auf dem konischen Endbereich 20 das Endstück 22 des Ausgaberohrs 24. Das Endstück 22 weist einen mit dem Endbereich 22 korrespondierenden Innenkonus auf. Die Kombination von Innenkonus, Außenkonus und Überwurfmutter bildet ein modifiziertes Luer-Lock-System.

Das in den Figuren 5 und 6 in Alleinstellung dargestellte Endstück 22 des Ausgaberohrs 24 weist, wie oben bereits erwähnt, einen Innenkonus 42 sowie an der dem Innenkonus abgewandten Seite 42 eine zylindrische Aufnahme 44 auf, in die ein Rohrabschnitt 46 des Ausgaberohrs 24 in Presspassung eingesteckt ist. Die zylindrische Ausnehmung 44 und der Bereich des Innenkonus 42 sind über eine Öffnung 48 verringerten Durchmessers miteinander verbunden.

An der der zylindrischen Aufnahme 44 abgewandten Stirnseite weist das Endstück 22 des Aüsgaberohrs 26 die oben bereits genannten radial nach außen vorstehenden Laschen 28 auf, die mit der in den Figuren 7 und 8 detailliert dargestellten Überwurfmutter 18 zusammenwirken.

Die Überwurfmutter 18 weist einen Gewindeabschnitt 50 auf, in dem das Innengewinde 30 ausgebildet ist. An der zu dem Gewindeabschnitt 50 abgewandten Seite weist die Überwurfmutter 18 einen Führungsabschnitt 52 auf, der auf dem Ringbund 34 des Adapters 16 geführt ist.

Der Gewindeabschnitt 50 und der Führungsabschnitt 52 sind über eine Trennwand 54 voneinander getrennt, die mit einer Öffnung 56 versehen ist, deren Rand die Überwurfmutter 18 im Bereich der Ringnut 40 des Adapters 16 führt.

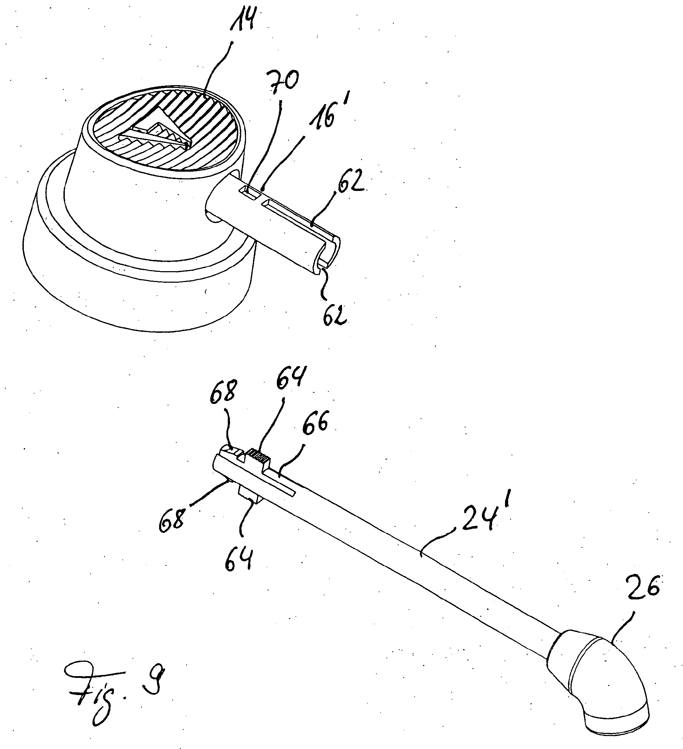

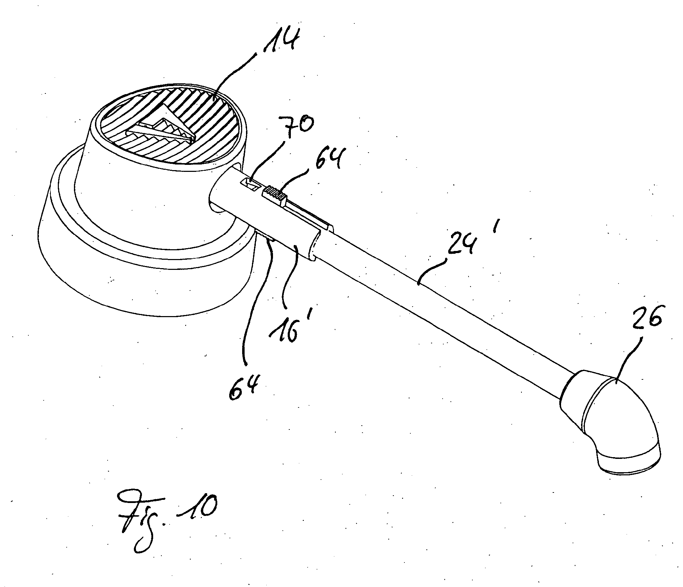

Die in den Figuren 9 und 10 dargestellte Ausführüngsform umfasst ebenfalls einen Sprühkopf 14, der eine radiale Öffnung 58 aufweist, in der über eine Presspassung ein Adapter 16' eingesetzt ist.

Der Adapter 16' weist hierzu einen Endabschnitt 60 verringerten Durchmessers auf.

An seiner, dem Endabschnitt 60 abgewandten Seite ist der röhrchenförmige Adapter 16' mit axialen Schlitzen 62 versehen, die um 180° zueinander versetzt sind und als Führungsbahnen für Betätigungstaster 64 von endseitigen Rastlaschen 66 eines auswechselbaren Ausgaberohrs 24' dienen. Die Rastlaschen 66 sind jeweils durch zwei axiale Schlitze 72 des Ausgaberohrs 24' gebildet.

Die Rastlaschen 66 weisen in ihren Endabschnitten radial vorstehende Rastnasen auf, die zur Sicherung des Ausgaberohrs 24' an dem Adapter 16 in Ausnehmungen 70 des Adapters 16' eingreifen.

Zum Lösen des Ausgaberohrs 24' von dem Adapter 16' bzw. zum Wechseln des Ausgaberohrs 24' werden die in den Führungsschlitzen 62 angeordneten Betätigungstaster 64 gedrückt, so dass die Rastnasen 68 der Rastlaschen 66 aus den Ausnehmungen 70 gedrückt werden und das Ausgaberohr 24' aus dem Adapter 16' gezogen werden kann. Dann kann ein neues hygienisch einwandfreies Ausgaberohr 24' in den Adapter 16' eingesetzt und über deren Rastlaschen an dem Adapter 16' gesichert werden.

### Bezugszeichen

- 10: Sprühdose
- 12: Dosenkörper
- 14: Sprühkopf
- 16: Adapter
- 18: Überwurfmutter
- 20: Endbereich
- 22: Endstück
- 24: Ausgaberohr
- 26: Düsenkopf
- 28: Laschen
- 30: Innengewinde
- 32: Endbereich
- 34: Ringfläche
- 36: Stirnfläche
- 38: Stirnfläche
- 40: Ringnut
- 42: Innenkonus
- 44: Aufnahme
- 46: Rohrabschnitt
- 48: Öffnung
- 50: Gewindeabschnitt
- 52: Führungsabschnit
- 54: Trennwand
- 56: Öffnung
- 58: Öffnung
- 60: Endabschnitt
- 62: Schlitz
- 64: Betätigungstaste
- 66: Rastlasche
- 68: Rastnase
- 70: Ausnehmungen
- 72: Schlitz

## Patentansprüche

1. Sprühdose, mit einem Dosenkörper (12) zur Aufnahme eines zu versprühenden Stoffes, einem Sprühkopf (14), der auf ein an der Oberseite des Dosenkörpers (12) angeordnetes Dosenventil aufgesteckt ist und der mit einem Ausgaberohr (24) versehen ist, wobei ein Adapter den Sprühkopf (14) mit dem Ausgaberohr verbindet, wobei das Ausgaberohr (24) über eine Schraubverbindung mit dem Adapter (16) verbunden ist, **gekennzeichnet durch** eine Sicherungseinrichtung, die das Ausgaberohr (24) an dem Adapter (16) sichert und zum Trennen des Ausgaberohrs (24) von dem Adapter (16) lösbar ist und eine an dem Adapter (16) gelagerte Überwurfmutter (18) umfasst, die mit einem Gegenstück (22) zusammenwirkt, das an dem Ausgaberohr (24) angeordnet ist.

2. Sprühdose nach Anspruch 1, **dadurch gekennzeichnet, dass** die Schraubverbindung einen Außenkonus umfasst, der an dem Adapter (16) oder an dem Ausgaberohr (24) ausgebildet ist und der mit einem Innenkonus des Ausgaberohrs (24) bzw. des Adapters (16) zusammenwirkt, so dass zwischen dem Adapter (16) und dem Ausgaberohr (24) eine Presspassung vorliegt.

3. Sprühdose nach Anspruch 1 oder 2, **dadurch gekennzeichnet, dass** der Adapter (16) eine Ringnut (40) aufweist, in der die Überwurfmutter (18) gelagert ist.

4. Sprühdose nach Anspruch 3, **dadurch gekennzeichnet, dass** die Ringnut (40) so ausgebildet ist, dass die Überwurfmutter (18) axial verschiebbar in ihr gelagert ist.

5. Sprühdose nach Anspruch 2, **dadurch gekennzeichnet, dass** der Innenkonus oder der Außenkonus einstückig mit dem Ausgaberohr (24) gefertigt ist.

6. Sprühdose nach Anspruch 2, **dadurch gekennzeichnet, dass** der Innenkonus oder der Außenkonus an einem Endstück (22) des Ausgaberohrs (24) ausgebildet ist.

7. Sprühdose, mit einem Dosenkörper (12) zur Aufnahme eines zu versprühenden Stoffes, einem Sprühkopf (14), der auf ein an der Oberseite des Dosenkörpers (12) angeordnetes Dosenventil aufgesteckt ist und der mit einem Ausgaberohr (24) versehen ist, wobei ein Adapter den Sprühkopf (14) mit dem Ausgaberohr verbindet, wobei das Ausgaberohr (24) über eine Steckverbindung mit dem Adapter (16) verbunden ist, wobei das Ausgaberohr (24') an seinem dem Adapter (16') zugewandten Ende mindestens eine Rastlasche (64) aufweist, die mit einem Rastelement (68) in eine Ausnehmung (70) des Adapters (16') eingreift, wobei die Rastlasche (66) ein in radialer Richtung vorstehendes Betätigungselement (64) aufweist, **dadurch gekennzeichnet, dass** das Ausgaberohr (24') in einen Kanal des Adapters (16') eingesteckt ist und der Adapter (16') eine Führungsbahn (62) für das Rastelement (68) aufweist, und dass das Betätigungselement (64) beim Einstecken des Ausgaberohrs (24') in den Adapter (16') in der Führungsbahn (62) geführt ist.

8. Sprühdose nach Anspruch 7, **dadurch gekennzeichnet, dass** die Rastlasche (66) durch axiale Schlitze (72) des Ausgaberohrs (24') gebildet ist.

9. Sprühdose nach Anspruch 7, **dadurch gekennzeichnet, dass** der Kanal eine mit einem Dichtmittel versehene Innenwand aufweist.

10. Sprühdose nach einem der Ansprüche 1 bis 9, **dadurch gekennzeichnet, dass** der Adapter (16) über eine Presspassung mit dem Sprühkopf (14) verbunden ist.

11. Sprühdose nach einem der Ansprüche 1 bis 10, **dadurch gekennzeichnet, dass** der Adapter und ein Gehäuse des Sprühkopfes einstückig hergestellt sind.

12. Sprühdose nach einem der Ansprüche 1 bis 11, **dadurch gekennzeichnet, dass** das Ausgaberohr (24) an seinem dem Sprühkopf (14) abgewandten Ende mit einem Düsenkopf (26) versehen ist.

## Claims

1. A spray can having a can body (12) for receiving a substance to be sprayed, a spray head (14) that is placed onto a can valve arranged at the upper side of the can body (12) and that is provided with a discharge tube (24), wherein an adapter connects the spray head (14) to the discharge tube, wherein the discharge tube (24) is connected to the adapter (16) via a screw connection, **characterized by** a securing device that secures the discharge tube (24) at the adapter (16) and is releasable to separate the discharge tube (24) from the adapter (16) and that comprises a cap nut (18) that is supported at the adapter (16) and that cooperates with a counter-piece (22) that is arranged at the discharge tube (24).

2. A spray can in accordance with claim 1, **characterized in that** the screw connection comprises an outer cone that is formed at the adapter (16) or at the discharge tube (24) and that cooperates with an inner cone of the discharge tube (24) or of the adapter (16) such that a press fit is present between the adapter (16) and the discharge tube (24).

3. A spray can in accordance with claim 1 or claim 2, **characterized in that** adapter (16) has an annular groove (40) in which the cap nut (18) is supported.

4. A spray can in accordance with claim 3, **characterized in that** the annular groove (40) is configured such that the cap nut (18) is axially displaceable therein.

5. A spray can in accordance with claim 2, **characterized in that** the inner cone or the outer cone is produced in one piece with the discharge tube (24).

6. A spray can in accordance with claim 2, **characterized in that** the inner cone or the outer cone is formed at an end piece (22) of the discharge tube (24).

7. A spray can having a can body (12) for receiving a substance to be sprayed, a spray head (14) that is placed onto a can valve arranged at the upper side of the can body (12) and that is provided with a discharge tube (24), wherein an adapter connects the spray head (14) to the discharge tube, wherein the discharge tube (24) is connected to the adapter (16) via a plug-in connection, wherein the discharge tube (24) has at least one latching tab (64) at its end facing the adapter (16'), said latching tab engaging with a latching element (68) into a cut-out (70) of the adapter (16'), and wherein the latching tab (66) has an actuation element (64) projecting in the radial direction, **characterized in that** the discharge tube (24') is inserted into a passage of the adapter (16') and the adapter (16') has a guide path (62) for the latching element (68); and **in that** the actuation element (64) is guided in the guide path (62) on the insertion of the discharge tube (24') into the adapter (16').

8. A spray can in accordance with claim 7, **characterized in that** the latching tab (64) is formed by axial slits (72) of the discharge tube (24').

9. A spray can in accordance with claim 7, **characterized in that** the passage has an inner wall provided with a sealant.

10. A spray can in accordance with any one of the claims 1 to 9, **characterized in that** the adapter (16) is connected to the spray head (14) via a press fit.

11. A spray can in accordance with any of the claims 1 to 10, **characterized in that** the adapter and a housing of the spray head are manufactured in one piece.

12. A spray can in accordance with any one of the claims 1 to 11, **characterized in that** the discharge tube (24) is provided with a nozzle head (26) at its end remote from the spray head (14).

## Revendications

1. Canette à pulvérisateur, comprenant un corps de canette (12) pour loger un produit à pulvériser, une tête de pulvérisation (14), qui est emboîtée sur une soupape de canette agencée sur la face supérieure du corps de canettes (12) et qui est dotée d'un tube distributeur (24), dans laquelle un adaptateur relie la tête de pulvérisation (14) au tube distributeur, et le tube distributeur (24) est relié à l'adaptateur (16) via une liaison vissée,
**caractérisée par** un moyen de blocage, qui bloque le tube distributeur (24) sur l'adaptateur (16) et qui est libérable pour séparer le tube distributeur (24) vis-à-vis de l'adaptateur, et inclut un écrou-collier (18) monté sur l'adaptateur (16), ledit écrou-collier coopérant avec une pièce antagoniste (22) qui est agencée sur le tube distributeur (24).

2. Canette à pulvérisateur selon la revendication 1, **caractérisée en ce que** la liaison vissée inclut un cône extérieur, qui est réalisé sur l'adaptateur (16) ou sur le tube distributeur (24) et qui coopère avec un cône intérieur du tube distributeur (24) ou respectivement de l'adaptateur (16), de sorte qu'il se présente un ajustement à pressage entre l'adaptateur (16) et le tube distributeur (24).

3. Canette à pulvérisateur selon la revendication 1 ou 2, **caractérisée en ce que** l'adaptateur (16) comporte une gorge annulaire (40) dans laquelle est monté l'écrou-collier (18).

4. Canette à pulvérisateur selon la revendication 3, **caractérisée en ce que** la gorge annulaire (40) est réalisée de telle façon que l'écrou-collier (18) est monté dans celle-ci avec possibilité de déplacement axial.

5. Canette à pulvérisateur selon la revendication 2, **caractérisée en ce que** le cône intérieur ou le cône extérieur est réalisé d'une seule pièce avec le tube distributeur (24).

6. Canette à pulvérisateur selon la revendication 2, **caractérisée en ce que** le cône intérieur ou le cône extérieur est réalisé sur une pièce terminale (22) du tube distributeur (24).

7. Canette à pulvérisateur comprenant un corps de canette (12) pour loger un produit à pulvériser, une tête de pulvérisation (14), qui est emboîtée sur une soupape de canette agencée sur la face supérieure du corps de canette (12) et qui est pourvue d'un tube distributeur (24), dans laquelle un adaptateur relie la tête de pulvérisation (14) au tube distributeur, et le tube distributeur (24) est relié à l'adaptateur (16) via une liaison à enfichage, dans laquelle le tube distributeur (24') comporte, à son extrémité tournée vers l'adaptateur (16'), au moins une patte d'enclenchement (64), qui s'engage avec un élément d'enclenchement (68) jusque dans un évidement (70) de l'adaptateur (16'), ladite patte d'enclenchement (66) comportant un élément d'actionnement (64) qui dépasse en direction radiale,
**caractérisée en ce que** le tube distributeur (24') est emboîté dans un canal de l'adaptateur (16'), et l'adaptateur (16') comporte une voie de guidage (62) pour l'élément d'enclenchement (68), et **en ce que** l'élément d'actionnement (64) est guidé dans la voie de guidage (62) lors de l'emboîtement du tube distributeur (24') dans l'adaptateur (16').

8. Canette à pulvérisateur selon la revendication 7, **caractérisée en ce que** la patte d'enclenchement (66) est formée par des fentes axiales (72) du tube distributeur (24').

9. Canette à pulvérisateur selon la revendication 7, **caractérisée en ce que** le canal comporte une paroi intérieure dotée d'un moyen d'étanchéité.

10. Canette à pulvérisateur selon l'une des revendications 1 à 9, **caractérisée en ce que** l'adaptateur (16) est relié à la tête de pulvérisation (14) via un ajustement à pressage.

11. Canette à pulvérisateur selon l'une des revendications à 10, **caractérisée en ce que** l'adaptateur et un boîtier de la tête de pulvérisation sont fabriqués d'une seule pièce.

12. Canette à pulvérisateur selon l'une des revendications 1 à 11, **caractérisée en ce que** le tube distributeur (24) est doté d'une tête de buse (26) à son extrémité détournée de la tête de pulvérisation (14).
